# EUROPEAN PATENT APPLICATION

(11) **EP 4 793 927 A1**
(43) Date of publication of application: **19.08.2026**
(21) Application number: 24877165.1
(22) Date of filing: 08.10.2024
(51) Int. Cl.: G09B 23/28, A61B 1/00, G09B 9/00

(54) **MEDICAL SIMULATOR AND TRAINING METHOD USING MEDICAL SIMULATOR**

(30) Priority: 11.10.2023 JP 2023176230
(71) Applicant: R Zero Inc., Yonago-shi Tottori, 683-0823 (JP); Japan Technical Management Co., Ltd., Kurayoshi-shi Tottori, 682-0024 (JP)
(72) Inventor: FUJII, Masashi, Yonago-shi Tottori 683-0823 (JP); MATSUOKA, Masaaki, Yonago-shi Tottori 683-0823 (JP); KIMURA, Katsunori, Yonago-shi Tottori 683-0823 (JP); FUKUDA, Tomoharu, Yonago-shi Tottori 683-0823 (JP); KODANI,Yuta, Yonago-shi Tottori 683-0823 (JP); MISU, Hiroyuki, Yonago-shi Tottori 683-0823 (JP)
(74) Representative: Stöckeler, Ferdinand
(86) International application number: PCT/JP2024/035905
(87) International publication number: WO 2025/079570

(57) **Abstract**

The present invention provides a medical simulation and a training method using a medical simulator capable of efficient training by guiding an operation of an endoscope in endoscopic training using the medical simulator. In addition, the present invention provides a medical simulator that is as small and low-cost as possible so as to facilitate training at the actual site, and a training method using the medical simulator.

A medical simulator 10 includes a stomach part 6, which is a hollow organ model whose inside is imaged by a distal end 221 of an endoscope 22, with first to eleventh guide parts G1 to G11 arranged to correspond to different first to eleventh specific sites P1 to P11 being provided in the stomach part 6 to guide the endoscope 22.

## Description

### Technical Field

The present invention relates to a medical simulator and a training method using the medical simulator. Specifically, the present invention relates to a medical simulator for educational training using a hollow organ model whose inside is imaged by an endoscope of an endoscopic system to practice procedures that are close to actual ones, and a training method using the medical simulator.

### Background Art

Endoscopic systems are widely used at medical sites, and many types of endoscopic systems are provided depending on hollow organs and purposes of use, such as an upper gastrointestinal tract endoscope, a colon endoscope, a bronchoscope, a thoracoscope, and a vascular endoscope. This endoscopic system mainly includes a video scope (endoscope) equipped with an imaging element at the distal end, a system main body including a video system that converts an electric signal from the endoscope into a video signal, a light source device, etc., and a monitor onto which the video signal is projected from the system main body. In addition, some endoscopic systems can not only view images in organs but also enable tissue collection, polypectomy, etc.

An examination using the endoscopic system requires advanced skills in endoscopic procedures, because it is necessary to insert an insertion portion of an endoscope into an organ without damaging the inside of the organ and to discover all lesions without omission. Therefore, a medical simulation as in Patent Literature 1 is known, and a medical simulator using a hollow organ model for educational training on the insertion of endoscopes as in Non Patent Literature 1 is also commercially available.

Then, in order to improve endoscopic procedure skills using such a medical simulator, Patent Literature 2 describes a medical simulator in which a plurality of specific sites are set, and a lamp corresponding to a specific site that the distal end of the endoscope has reached is displayed by detecting light emission from an endoscope.

In addition, Patent Literature 3 discloses a medical simulator that performs image processing by machine learning or the like using endoscopic images acquired by an endoscope before implementation of educational training, acquires a position of the endoscope during training on the basis of the image processing data, further adds guide information to the endoscopic image during implementation of educational training, and displays the endoscopic image on a display device constituting the medical simulator.

### Citation List

### Patent Literature

Patent Literature 1: JP 7339679 B2
Patent Literature 2: JP 61-213877 A
Patent Literature 3: JP 6632020 B1

### Non Patent Literature

Non Patent Literature 1: R0 Corporation "Next Generation Medical Simulator "mikoto Colon Endoscope Model" Search Date: September 25, 2023
<https://rzero.jp/mikoto/index.html>

### Summary of Invention

### Technical Problem

However, in the medical simulator of Patent Literature 2, if the trainee's level of skill is low, the trainee does not even know where the specific sites are in the first place, and as a result, there is a problem that the trainee ends up searching for the specific sites by hand in a blind way or relying on an instructor for guidance during operation.

In addition, when image processing is performed using an endoscopic image as in Patent Literature 3, complicated image processing is required, which increases the cost of medical simulators, and hinders the widespread use of medical simulators.

In addition, training using a medical simulator may be performed in a dedicated training room, but is often performed in an actual site such as an examination room in a hospital. In such a case, a trainee is trained using an endoscopic system that is being used at the site, with a medical simulator brought to the site. In such an actual training environment, it is practically difficult to install a large-scale medical simulator including a large dedicated display device as in Patent Literature 3 at the site. Therefore, under such a training environment, it is preferable that training can be performed only with an endoscopic system installed at the site and a medical simulator that is as small as possible.

Therefore, the present invention provides a medical simulator capable of efficient training by guiding an operation of an endoscope in training using the medical simulator, and a training method using the medical simulator. In addition, the present invention provides a medical simulator that is as small and low-cost as possible so as to facilitate training at the actual site, and a training method using the medical simulator.

### Solution to Problem

In order to achieve the aforementioned object, a medical simulator according to the present invention is a medical simulator including a hollow organ model whose inside is imaged by an endoscope, in which a plurality of guide parts arranged at different specific sites are provided in the hollow organ model.

In the medical simulator according to the present invention, since the guide part is provided in the hollow organ model, a trainee can operate the endoscope by aiming at the guide part imaged by the endoscope, thereby enabling the trainee to efficiently learn endoscopic procedures. For example, if a guide part is provided at a specific site that needs to be checked, the trainee can efficiently learn the position of the specific site. In addition, if guide parts are provided in the order in which they are to be checked, the trainee can efficiently learn the order in which the endoscope should be maneuvered.

In addition, since the guide part itself is provided in the hollow organ model, the guide part can be recognized from the image itself captured by the endoscope, and thus can be displayed on the monitor itself of the endoscope system. Since the medical simulator of the present invention does not need to perform advanced image processing and does not need a large dedicated display device for displaying guide information unlike the medical simulator of Patent Literature 3, it is possible to realize a small and low cost medical simulator.

Further, such a medical simulator according to the present invention can also be expressed as a medical simulator including a hollow organ model whose inside is imaged by an endoscope, in which a guide part is disposed in the hollow organ model, and the guide part is configured such that the position where the guide part is disposed is projected by capturing an image using the endoscope.

Note that such a guide part can be realized, for example, by a mark or a number serving as a marker given to a specific site on the inner surface of the hollow organ model in addition to the guide part to be described later.

In addition, in the medical simulator according to the present invention, it is preferable that the guide part is a light emitter, and the medical simulator further includes a guide control unit that controls light emission of the light emitter.

In the medical simulator according to the present invention, since the guide part is a light emitter, for example, a specific site that needs to be checked is illuminated by the light emitter, and thus, the specific site is illuminated and projected onto an image captured by the endoscope. Therefore, unlike a guide part formed of a mark or a number, the trainee can very easily recognize the guide part inside the complicated hollow organ model that is difficult to observe.

Furthermore, the guide control unit that controls light emission enables easy settings of, for example, the order in which the trainer guides the trainee to check the specific sites and the specific site desired to be reached by the endoscope by selecting the light emitter, setting the order in which the light emitters are to be turned on, selecting whether to turn on/blink/turn off the light emitter.

In the medical simulator according to the present invention, it is preferable that the hollow organ model is made of a light-transmissive material, each of the guide parts includes a light emitter and an illuminance sensor attached to an outer surface of the hollow organ model, and the medical simulator further includes a guide control unit that controls light emission of the light emitter and light reception of the illuminance sensor.

In the medical simulator according to the present invention, the light emission of the guide part enables the trainee to easily recognize the guide part. In addition, the illuminance sensor detects light emission from the illumination lens at the distal end of the endoscope, thereby making it possible to detect that the position of the distal end of the endoscope has reached the guide part.

That is, in the medical simulator according to the present invention, the guide part of the hollow organ model can provide a marker for operating the endoscope through the light emitter and detect the position of the distal end of the endoscope through the illuminance sensor. Therefore, by using the medical simulator according to the present invention, for example, when the endoscope reached the first specific site where the light emitter emits light, the light emitter of the next second specific site can be turned on, so that the trainee can efficiently learn the order in which the specific sites are to be checked. Furthermore, for example, it is also possible to realize training in which the light emitters of all the guide parts are turned on first, and then the light emitters are sequentially turned off from the guide part that the endoscope has reached.

In addition, since the guide part is attached to the outer surface of the hollow organ model, the guide part is not directly exposed to the surface in the hollow organ model, so that the medical simulator according to the present invention can realistically reproduce the inside of the hollow organ model.

In the medical simulator according to the present invention, it is preferable that the guide part is detachably attached via a guide attachment part provided on the outer surface of the hollow organ model.

In the medical simulator according to the present invention, since the guide part is detachable, when the guide part breaks down, it is not necessary to replace the entire hollow organ model, and only the broken guide part can be replaced. Therefore, the present invention can provide a medical simulator with lower cost. In addition, in the present invention, it is easy to replace the light emitter and the illuminance sensor with different types.

It is preferable that the guide attachment part further includes a flat portion that receives the guide part.

This is because it is usual in the hollow organ model that irregularities corresponding to the irregularities on the inner surface are also formed on the outer surface. Therefore, if the guide part is directly attached to the outer surface, the guide part may become unstable due to the irregularities on the outer surface, which may greatly affect the reception of light by the illuminance sensor. However, in the medical simulator according to the present invention, the flat portion makes the attached state of the guide part very stable.

In addition, since light emission from the illumination lens at the distal end of the endoscope has relatively strong directivity, it may be difficult for the illuminance sensor to receive light even if the distal end of the endoscope is located near the guide part depending on the orientation of the distal end of the endoscope. However, in the medical simulator according to the present invention, light emission from the illumination lens is diffused to some extent in the flat portion, stabilizing detection by the illuminance sensor.

A training method using a medical simulator according to the present invention is an endoscope training method using a medical simulator including a hollow organ model whose inside is imaged by an endoscope, in which a plurality of guide parts arranged at different specific sites are provided in the hollow organ model to guide the endoscope, the hollow organ model is made of a light-transmissive material, the guide part includes a light emitter and an illuminance sensor attached to an outer surface of the hollow organ model, the light emitter intermittently emits light, and the illuminance sensor is in a light receivable state when the light emitter is turned off.

In the training method using the medical simulator according to the present invention, in the guide part including the light emitter and the illuminance sensor, light emission from the illumination lens at the distal end of the endoscope can be detected by the illuminance sensor when the light emitter is turned off, thereby making it possible to reliably detect that the position of the distal end of the endoscope reached the guide part without erroneous detection in which light emission from the light emitter is erroneously detected by the illuminance sensor. That is, endoscopic procedure training can be performed by combining the marker using the light emitter and the detection of the position of the distal end of the endoscope using the illuminance sensor.

As the endoscopic procedure training, various types can be realized. For example, the training is performed in such a manner that, when the endoscope reaches the first specific site where the light emitter emits light, the light emitter of the next second specific site is turned on. In addition, the training is performed in such a manner that the light emitters of all the guide parts are turned on first, and then the light emitters are sequentially turned off from the guide part that the endoscope has reached.

Then, in such training, the time required for the training may be measured using a clock function provided in the medical simulator or a separately prepared clock.

### Brief Description of Drawings

Fig. 1A is a conceptual diagram of training using a medical simulator according to the present embodiment, and Fig. 1B is an external view of the medical simulator.
Fig. 2A is a view illustrating an internal state of the medical simulator, and Fig. 2B is an enlarged view illustrating a stomach part accommodated in the medical simulator.
Fig. 3 is a block diagram illustrating main components of the medical simulator according to the present embodiment.
Fig. 4 is a view illustrating specific sites in the stomach part.
Fig. 5 is an exploded view illustrating a configuration of a guide part according to the present embodiment.
Fig. 6A is an image of the guide part attached to the stomach part, and Fig. 6B is a cross-sectional view of the guide part attached to the stomach part.
Fig. 7 is a time chart illustrating timings at which a light emitter and an illuminance sensor operate in the guide part according to the present embodiment.
Fig. 8 is a flowchart illustrating an operation of a guide control unit in a training method using the medical simulator according to the present embodiment.

### Description of Embodiments

Hereinafter, a mode for carrying out the present invention will be described with reference to the embodiment and drawings, but the following embodiment is not intended to limit the present invention to what is described herein, and the present invention can be equally applied to various modifications without departing from the technical idea set forth in the claims. In each drawing used for explanation in this specification, each member is shown at a different scale in order to make each member recognizable in the drawing, and is not necessarily shown in proportion to its actual dimension.

### [Embodiment]

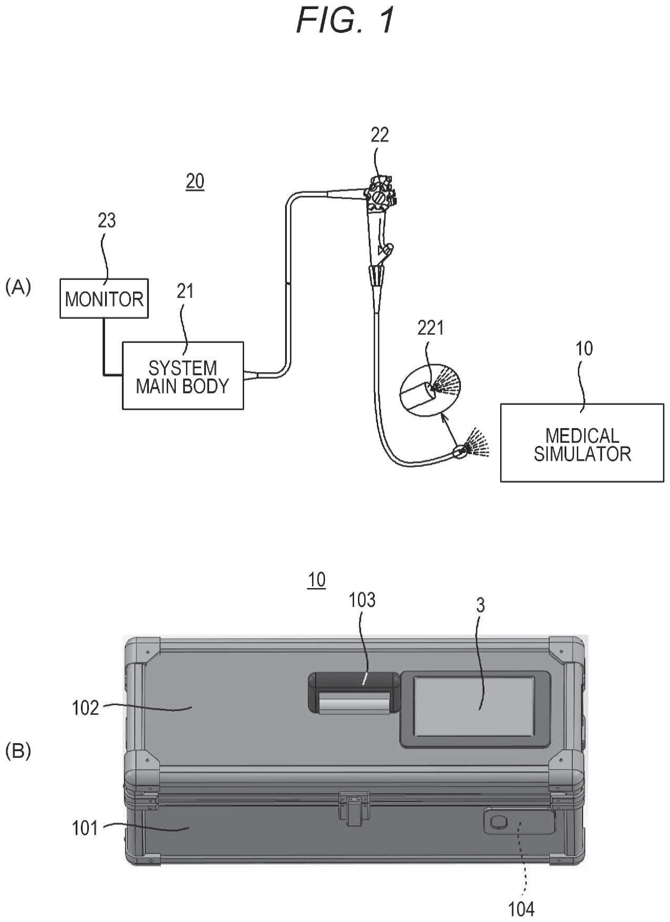

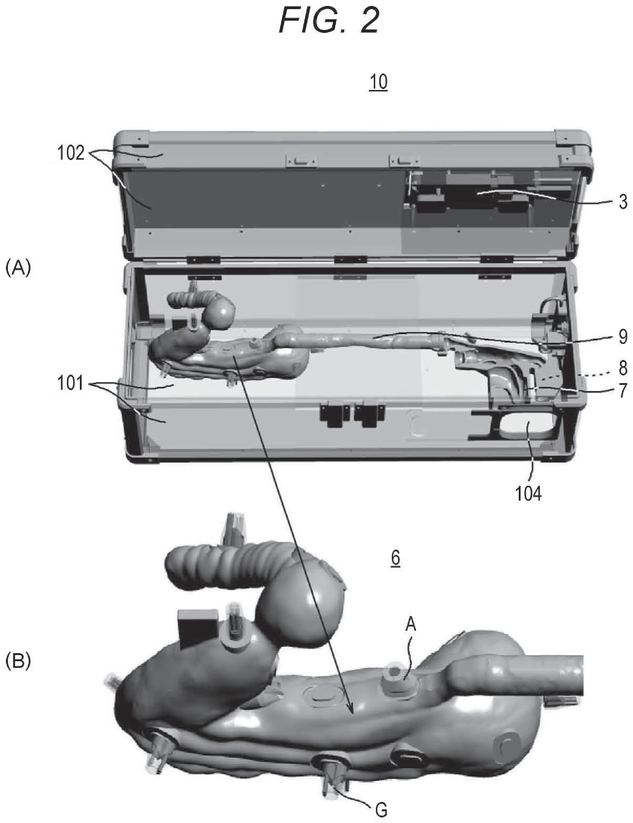

A medical simulator 10 according to the present embodiment will be described with reference to the drawings. Fig. 1A is a conceptual diagram of endoscopic procedure training using the medical simulator 10, and Fig. 1B is an external view of the medical simulator 10. Fig. 2A is a diagram illustrating an internal state of the medical simulator 10, and Fig. 2B is an enlarged view of a stomach part 6 accommodated in the medical simulator 10.

As illustrated in Fig. 1A, the medical simulator 10 is used with an endoscopic system 20. Here, the endoscopic system 20 is an existing system, and is installed in an examination room or the like in a hospital. Then, the endoscopic system 20 mainly includes a system main body 21 having a video system and a light source device, an endoscope 22 having an imaging element mounted on a distal end 221 thereof, and a monitor 23 onto which a video signal is projected from the system main body 21. At the distal end 221 of the endoscope 22, there are an objective lens for imaging (not illustrated), a plurality of illumination lenses for lighting, a suction/forceps port, an air/water supply nozzle, etc. Then, the light guided from the light source device of the system main body 21 illuminates an object through the illumination lens, the illuminated object is imaged by the imaging element through the objective lens, the electric signal is converted into a video signal by the video system of the system main body 21, and the video signal is displayed on the monitor 23.

The medical simulator 10 used for procedure training together with such an endoscopic system 20 includes a hollow organ model whose inside is imaged by the endoscope 22. More specifically, the medical simulator 10 has an openable and closable box-shaped external appearance, and accommodates a hollow organ model inside the box-shaped structure.

Note that the hollow organ model of the medical simulator 10 according to the present embodiment is specifically composed of an upper gastrointestinal tract having a stomach part 6 simulating a stomach as illustrated in Fig. 2B. Therefore, the medical simulator 10 according to the present embodiment is a medical simulator for upper gastrointestinal tract endoscopy training.

As illustrated in Fig. 1B, the medical simulator 10 having such an openable and closable box-shaped external appearance mainly includes an accommodating main body 101 and an openable and closable upper lid 102, and a handle 103 is attached to the upper lid 102. Therefore, the medical simulator 10 is sized so that it can be carried by holding the handle 103.

In addition, as illustrated in Fig. 2A, an oral cavity part 7, a nasal cavity part 8, and an esophagus part 9, into which the endoscope 22 is inserted, are accommodated in the accommodating main body 101 together with the stomach part 6 inside the medical simulator 10. Then, the oral cavity part 7 and the nasal cavity part 8 communicate with the outside through an opening portion 104 provided on a side surface of the accommodating main body 101, and the insertion of the endoscope 22 begins from the opening portion 104. Note that the opening portion 104 provided on the side surface of the accommodating main body 101 has a slide structure with respect to the side surface, and is slid to open and close the oral cavity part 7 or the nasal cavity part 8 with respect to the outside.

In addition, not only when the medical simulator 10 is carried, but also when training is performed using the medical simulator 10, and the like, the medical simulator 10 according to the present embodiment is usually used in a state where the upper lid 102 is closed as illustrated in Fig. 1B.

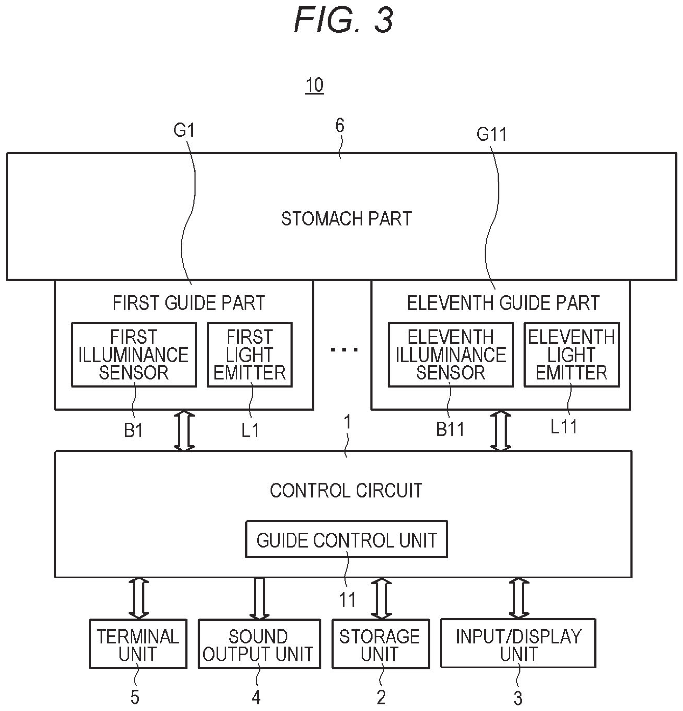

The main components of the medical simulator 10 will be described with reference to a block diagram illustrated in Fig. 3. The medical simulator 10 includes a control circuit 1 that controls the entire medical simulator 10, a storage unit 2 that stores programs executed by the control circuit 1, data necessary for operating the control circuit 1, and the like, an input/display unit 3 formed of a touch panel for performing start and stop operations and setting various conditions, a sound output unit 4 that emits sound and beep sound, a terminal unit 5 including an HDMI (registered trademark) port, a USB port, and the like, a stomach part 6 made of a light-transmissive flexible material, and first to eleventh guide parts G1 to G11 (collectively referred to as guide parts G) arranged on the outer surface of the stomach part 6.

In the present embodiment, the control circuit unit 1 and the storage unit 2 are provided together with the stomach part 6 in the accommodating main body 101, and the input/display unit 3 is provided on the surface of the upper lid 102 as illustrated in Fig. 1B, and is made of a relatively small display device with a touch panel of about 7 inches. Further, the sound output unit 4 and the terminal portion 5 are provided on the surface of the accommodating main body 101 or the upper lid 102.

Here, for the stomach part 6 made of a light-transmissive flexible material, a silicone material is specifically used. The stomach part 6 is produced by molding using a mold created based on three-dimensional information scanned using computed tomography (CT), nuclear magnetic resonance imaging (MRI), or the like, or by molding using a three-dimensional printer. In addition, since the silicone material inherently has excellent translucency and is easily colored with a pigment or the like, the silicone material is very suitable for creating the stomach part 6 having translucency as in the present embodiment.

Although the stomach part 6 is referred to, the stomach part 6 does not strictly mean only the stomach, which is a hollow organ, but also includes a part of the esophagus, which is a hollow organ, and a part of the duodenum, which is a hollow organ. In the present embodiment, the hollow organ model is a stomach, which is a hollow organ, but the hollow organ is not limited to the stomach. For example, the hollow organ model may be a gastrointestinal tract such as a large intestine, a small intestine, a gallbladder, or a bile duct, or a urinary tract system such as a ureter, a bladder, or a urethra.

The guide parts G are attached to the outer surface of the stomach part 6. The guide parts G are attached to correspond to positions of first to eleventh specific sites P1 to P11 (collectively referred to as specific sites P) of the stomach part 6.

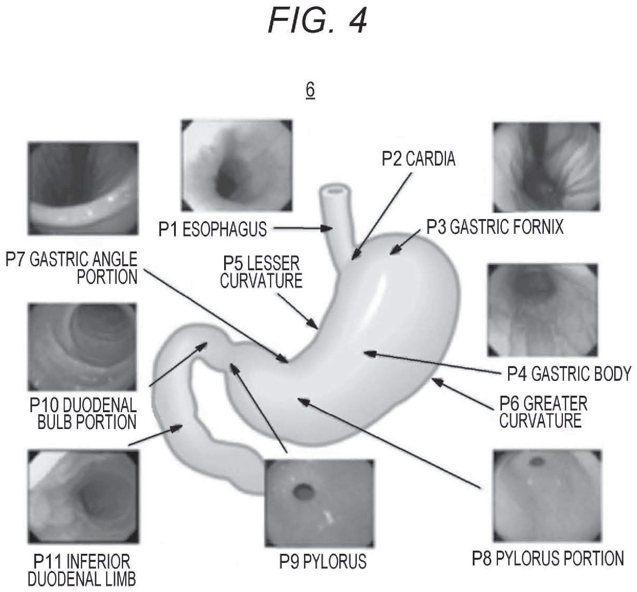

These specific sites P are important sites that should be reached and photographed by the endoscope 22 in actual upper gastrointestinal tract endoscopy inspections. Specifically, as illustrated in Fig. 4, an esophagus as the first specific site P1, a cardia as the second specific site P2, a gastric fornix (gastric fundus) as the third specific site P3, a gastric body portion as the fourth specific site P4, a lesser curvature as the fifth specific site P5, a greater curvature as the sixth specific site P6, a gastric angle portion as the seventh specific site P7, a pylorus portion as the eighth specific site P8, a pylorus as the ninth specific site P9, a duodenal bulb portion as the tenth specific site P10, and an inferior duodenal limb as the eleventh specific site P11 are set as main sites.

Note that, in the medical simulator 10 according to the present embodiment, the specific sites P of the stomach part 6 are the 11 specific sites P1 to P11 illustrated in Fig. 4, but the specific sites are not necessarily limited to these specific sites P, and may be fewer or more sites, or may be other sites. In addition, if the medical simulator uses a hollow organ model other than the stomach part 6, the specific sites will naturally differ.

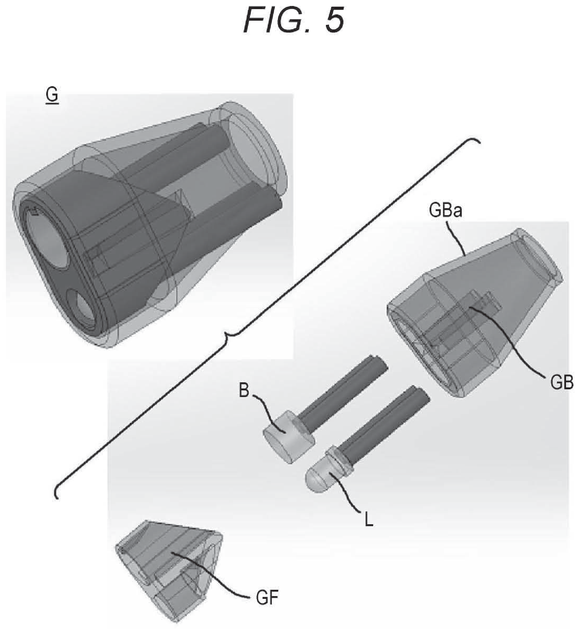

Then, as illustrated in Fig. 5, the guide part G attached to the outer surface of the stomach part 6 to correspond to the position of each specific site P according to the present embodiment includes a light emitter L that emits light using an LED light source and an illuminance sensor B that receives and detects irradiation light from the distal end 221 of the endoscope 22.

More specifically, in the guide part G, the light emitter L and the illuminance sensor B are held close to each other by a front fixture GF, and are integrated by a rear fixture GB that surrounds and holds the light emitter L, the illuminance sensor B, and the front fixture GF, and has openings through which wires connected to the light emitter L and the illuminance sensor B, respectively, are exposed. That is, the guide part G according to the present embodiment can also be referred to as a guide sensor including the light emitter L and the illuminance sensor B.

Note that the guide parts G corresponding to the first to eleventh specific sites P1 to P11 of the stomach part 6 are the first to eleventh guide parts G1 to G11, respectively, and the first to eleventh guide parts G1 to G11 include first to eleventh light emitters L1 to L11 and first to eleventh illuminance sensors B1 to B11, respectively.

As illustrated in Fig. 3, the guide part G including the light emitter L and the illuminance sensor B is controlled by a guide control unit 11 that also serves as the control circuit 1. The guide control unit 11 switches the light emitter L and the illuminance sensor B, sets the brightness detected by the illuminance sensor B, and the like. Then, when the illuminance of the endoscope 22 received by the illuminance sensor B becomes higher than a predetermined value, the guide control unit 11 determines that the endoscope 22 has reached the specific site P.

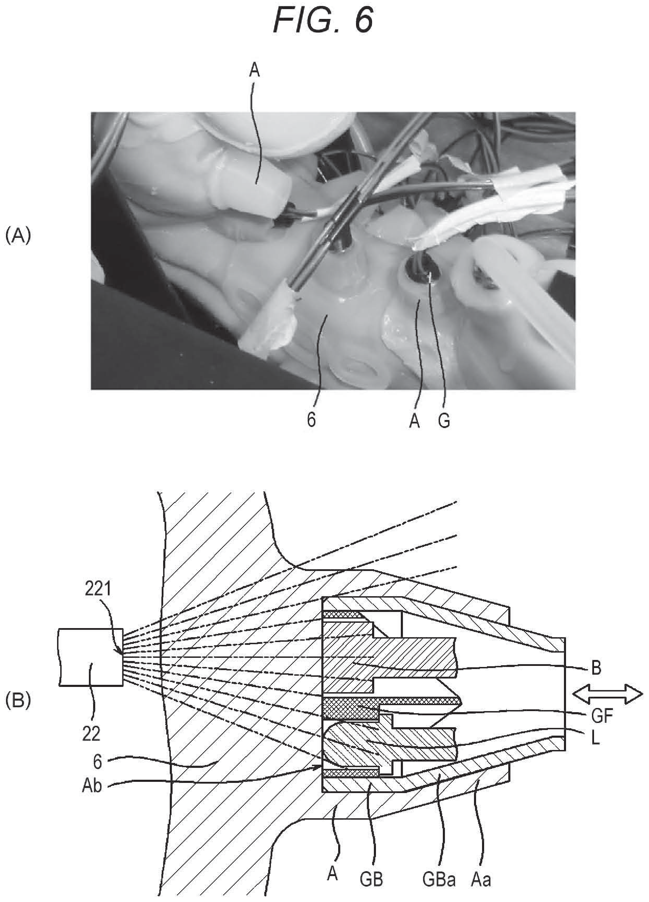

Such a guide part G is attached to the outer surface of the stomach part 6 as illustrated in Fig. 6. Fig. 6A is an image of the guide part G attached to the stomach part 6, and Fig. 6B is a cross-sectional view of the guide part G attached to the stomach part 6.

Specifically, a guide attachment part A having an elliptically cylindrical shape is formed on the outer surface of the stomach part 6 to correspond to the position of the specific site P of the stomach part 6. As illustrated in Fig. 6B, the guide attachment part A is formed integrally with the stomach part 6 formed of a light-transmissive flexible material. The guide attachment part A is slightly smaller than the guide part G. Therefore, by fitting the guide part G into the tube of the guide attachment part A, the guide part G is held by the stomach part 6.

That is, the guide attachment part A formed integrally with the stomach part 6 made of a flexible material stretches and expands by inserting the guide part G, and is deformed so as to follow the shape of an inclined portion GBa of the rear fixture GB of the guide part G, thereby forming an inclined portion Aa. Therefore, the guide part G is held so as not to come off easily. On the other hand, by strongly pulling the guide part G rearward, the inclined portion Aa of the guide attachment part A is deformed, and the guide part G can be pulled out from the guide attachment part A. In this manner, the guide part G is detachably attached to the outer surface of the stomach part 6 via the guide attachment part A.

Therefore, for example, if the guide part G breaks down, only the broken guide part G can be replaced, rather than replacing the entire stomach part 6. In addition, the light emitter L and the illuminance sensor B can be easily replaced with different types.

In addition, a flat portion Ab having a flat surface as an opposing surface for receiving the guide part G is further formed in the guide attachment part A. Therefore, the guide part G attached to the guide attachment part A is stably held. The flat portion Ab is not necessarily required to be formed, but since the stomach part 6 has, on the outer surface, irregularities corresponding to the irregularities on the inner surface, if the guide part G is directly attached to the outer surface, the guide part G may become unstable due to the irregularities on the outer surface. If the guide part G is attached unstably, this may greatly affect the reception of light by the illuminance sensor B. However, by attaching the guide part G via the guide attachment part A in which the flat portion Ab is formed as in the present embodiment, stable reception of light by the illuminance sensor B can be achieved.

In addition, when the distal end 221 of the endoscope 22 is positioned in the direction as illustrated in Fig. 6B, stable reception of light by the illuminance sensor B can be achieved. However, in practice, the distal end 221 of the endoscope 22 is rarely positioned in the direction as illustrated in Fig. 6B, and the distal end 221 may be positioned close to the light receiving surface of the illuminance sensor B in an oblique direction. Since the light emitted in the oblique direction from the position of the distal end 221 close in the oblique direction is diffused by the thickness of the flat portion Ab of the guide attachment part A, it is easy for the illuminance sensor B to receive the light. Therefore, it is preferable to form the flat portion Ab.

As described above, the medical simulator 10 according to the present embodiment includes a stomach part 6, which is a hollow organ model whose inside is imaged by the endoscope 22, and a guide part G is detachably attached to the position of each of the different specific sites P of the stomach part 6 via the guide attachment part A on the outer surface. The guide part G includes a light emitter L and an illuminance sensor B.

In such a medical simulator 10, since the stomach part 6 including the guide attachment part A is formed of a light-transmissive silicone material, the objective lens located at the distal end 221 of the endoscope 22 inserted into the stomach part 6 by a trainee can image light emission of the light emitter L of the guide part G in the stomach part 6. Therefore, the trainee can operate the endoscope 22 using the light emission of the light emitter L as a marker together with the inside of the stomach part 6 projected onto the monitor 23 of the endoscopic system 20.

In addition, since the stomach part 6 including the guide attachment part A is formed of a light-transmissive silicone material, the light emitted from the illumination lens located at the distal end 221 of the endoscope 22 inserted into the stomach part 6 by the trainee can be received by the illuminance sensor B of the guide part G. Therefore, the medical simulator 10 can detect that the distal end 221 of the endoscope 22 has reached the position of the corresponding guide part G as the illuminance sensor B receives light.

Therefore, for example, by turning off the light emitter L of the guide part G according to the detection of the illuminance sensor B, it is possible to cause the trainee who is operating the endoscope 22 while viewing the monitor 23 of the endoscopic system 20 to understand that the distal end 221 has reached the specific site P corresponding to the guide part G.

Furthermore, in the medical simulator 10 according to the present embodiment, the image captured by the endoscope 22 itself contains an image of the specific site P directly illuminated by the light emitter L of the guide part G. Therefore, by using the captured image, the specific site P directly illuminated by the light emitter L of the guide part G can be displayed on the monitor 23 of the endoscopic system 20. Therefore, unlike Patent Literature 3, it is not necessary to separately add guide information to the imaging of the endoscope 22. Therefore, it is not necessary to perform expensive image processing, and it is possible to provide an inexpensive and small medical simulator 10.

In addition, the medical simulator 10 according to the present embodiment is provided in the form of a portable box as illustrated in Fig. 1B. Then, as illustrated in Fig. 1A, the trainee can perform endoscopic procedure training using the small medical simulator 10 and the endoscopic system 20. Therefore, unlike the training described in Patent Literature 3, the medical simulator 10 can be brought into an actual site, such as an examination room in a hospital, so that training can be easily realized using the endoscopic system 20 used in the actual site.

In the guide part G including the light emitter L and the illuminance sensor B as in the present embodiment, the light emitter L and the illuminance sensor B are held close to each other as illustrated in Fig. 5. Therefore, it is conceivable that the light emitter L, which is originally used to receive light from the distal end 221 of the endoscope 22, detects light emission from the adjacent light emitter L. In particular, in a case where the guide part G is attached via the guide attachment part A, since the guide attachment part A is also formed of a light-transmissive silicone material, the light emission of the light emitter L is further diffused, so that the light is more easily received by the illuminance sensor B.

In the present embodiment, as illustrated in Figs. 5 and 6B, the light emitter L and the illuminance sensor B are arranged to be flush with each other. When the light emitter L and the illuminance sensor B are arranged as described above, the light emission from the light emitter L can be easily detected by the illuminance sensor B. Therefore, for example, by arranging the light receiving surface of the illuminance sensor B at a position retracted (in a direction away from the flat portion Ab) from the light emitting surface of the light emitter L, the light emission from the light emitter L can be hardly detected by the illuminance sensor B.

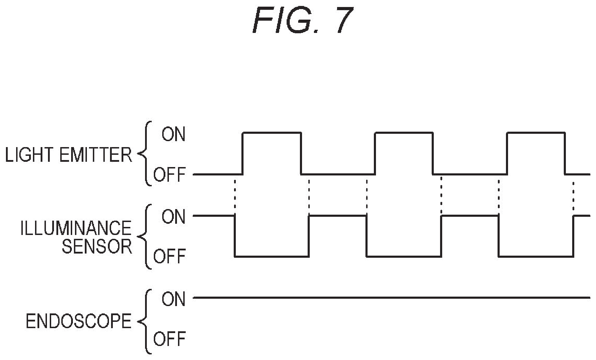

On the other hand, in the configuration of the guide part G in which the light emitter L and the illuminance sensor B are arranged to be flush with each other as in the present embodiment, the medical simulator 10 according to the present embodiment performs control as illustrated in Fig. 7 using the guide control unit 11. Specifically, the guide control unit 11 intermittently performs light emission of the light emitter L, performs measurement with the illuminance sensor B when the light emitter L is turned off (OFF), and detects the illuminance of the endoscope 22.

Therefore, since the illuminance sensor B detects light from the distal end 221 of the endoscope 22 when the light emitter L is turned off, in the configuration of the guide part G as in the present embodiment, detection of light from the endoscope 22 is not hindered by the adjacent light emitter L in the illuminance sensor B. In addition, in the configuration of the present embodiment, when the light emitter L of the guide part G functions to guide the distal end 221 of the endoscope 22 to the specific site P for the trainee, the light emitter L is in a blinking state in which the light emitter L is repeatedly turned on and off.

In this manner, in the medical simulator 10 according to the present embodiment, in the guide part G including the light emitter L and the illuminance sensor B, the light emitter L intermittently emits light under the control of the guide control unit 11, and the illuminance sensor B is in a light receivable state when the light emitter L is turned off.

Therefore, in the guide part G including the light emitter L and the illuminance sensor B, light emission from the illumination lens at the distal end 221 of the endoscope 22 can be detected by the illuminance sensor B when the light emitter L is turned off, thereby making it possible to reliably detect that the position of the distal end 221 of the endoscope 22 reaches the guide part G without erroneous detection in which light emission from the light emitter L is erroneously detected by the illuminance sensor B. That is, various types of endoscopic procedure training can be performed by combining the marker using the light emitter L and the detection of the position of the distal end 221 of the endoscope 22 using the illuminance sensor B.

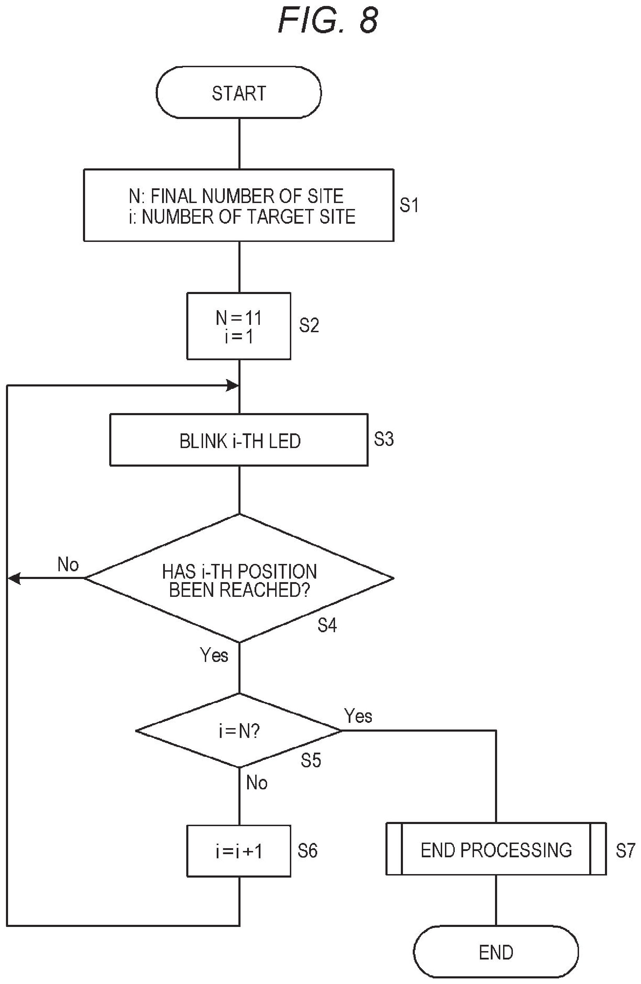

Next, an example of a specific training method using the medical simulator 10 according to the present embodiment will be described. Fig. 8 is a flowchart illustrating an operation in the guide control unit 11 of the medical simulator 10 when the trainee performs training to sequentially check the first to eleventh specific sites P1 to P11 of the stomach part 6.

As illustrated in Fig. 8, the guide control unit 11 sets a variable N as the final number of the specific site P, and defines a variable i as the number of the specific site P to be reached by the distal end 221 of the endoscope 22 (step S1). Then, the variable N is set to 11, and the initial value of the variable i is set to 1 (step S2). The guide control unit 11 blinks only the light emitter L of the specific site P in the order of the variable i (step S3). Then, it is determined whether the distal end 221 has reached the illuminance sensor B of the specific site P in the order i (step S4). When the distal end 221 has not reached the specific site P in the order i, the guide control unit 11 returns to step S3 (No in step S4).

When the distal end 221 of the endoscope 22 has reached the specific site P in the order i in step S4 (Yes in step S4), the guide control unit 11 determines whether the variable i is the final number N (step S5). That is, it is checked whether the reached specific site P is final. When the variable i is not the final number N, the guide control unit 11 adds one to the variable i (step S6) and returns to step S3 (No in step S5). That is, by adding one to the variable i, in step S3, only the light emitter L of the specific site P that is the next target to be reached is blinked to guide the endoscope to the next target position.

Then, when the variable i is the final number N in step S5 (Yes in step S5), the guide control unit 11 performs end processing (step S7).

As this end processing, various types of processing can be considered. For example, the end is displayed on the input/display unit 3, or is notified by sound through the sound output unit 4 to urge the removal of the endoscope 22. In addition, the performance result of the training may be displayed. For this performance result, for example, a time required for training is measured by a clock unit (not illustrated) provided in the medical simulator 10, and information based on the time can be used as a performance result. In addition, the procedure may be scored or ranked, and the information may be used as a performance result.

Furthermore, before starting the training as illustrated in Fig. 8, the input/display unit 3 or the sound output unit 4 of the medical simulator 10 may be used to reproduce a guidance video for explaining the training contents, explaining the endoscopic procedure, and the like.

As described above, in the training method using the medical simulator 10 according to the present embodiment, since the specific sites P1 to P11 to be reached by the endoscope 22 are sequentially guided by blinking the light emitters L, it is possible to observe all of the plurality of specific sites P without omission. Then, unlike Patent Literatures 2 and 3, when a target specific site P is reached, the light emitter L of the guide part G corresponding to the specific site P of the next target is automatically caused to emit light to provide navigation, and thus, it is particularly convenient for a beginner trainee, making it easy to recognize the target position.

Furthermore, in addition to the training method using the medical simulator 10 illustrated in Fig. 8, for example, training can be performed in which the guide control unit 11 first blinks all the light emitters L of the guide parts B corresponding to all the specific sites P, turns off the light emitters L of the guide parts B corresponding to the specific sites P from the specific site P that the distal end 221 of the endoscope 22 has reached, and measures the time until all the light emitters L are turned off.

In this manner, various types of training can be realized by using the medical simulator 10 including the guide part G including the light emitter L and the illuminance sensor B according to the present embodiment.

In addition, a certain trainer may provide guidance in such a manner that the order in which the first to eleventh specific sites P1 to P11 are checked may differ from this numerical order. However, in the medical simulator 10 according to the present embodiment, the guide control unit 11 can also easily perform control to cause the light emitters L to emit light in an order desired by a trainer who provides guidance. For example, the trainer or the trainee can set such control via the input/display unit 3.

In the medical simulator 10 according to the present embodiment, the guide part G includes the light emitter L and the illuminance sensor B. However, the medical simulator according to the present invention is not limited to such a guide part G. For example, marks or numbers serving as markers may be provided on the inner surface of the hollow organ model, and these marks or numbers may be used as guide parts. Even in such a configuration in which marks or numbers directly provided on the inner surface of the hollow organ model are used as guide parts, the guide parts in the hollow organ model can be projected by the endoscope 22. In addition, if the guide part has such a configuration, since the guide control unit and the guide attachment part are not required, it is possible to realize a medical simulator with lower cost.

In addition, it is also possible to adopt a configuration in which the guide part is made up of only the light emitter L, and only light emission control is performed by the guide control unit. Even in a configuration in which only such a light emitter is used as the guide part, the guide part in the hollow organ model can be projected by the endoscope 22. In addition, in particular, since the inside of the hollow organ model is very complicated, the marks and numbers as described above are likely to be hidden, but the markers using light emission are very easy for the trainee to recognize.

Furthermore, in the medical simulator 10 according to the present embodiment, the image captured by the endoscope 22 is projected onto the monitor 23 of the endoscopic system 20, but can also be projected onto the input/display unit 3 included in the medical simulator 10. In a case where such image data captured by the endoscope 22 is acquired by the medical simulator 10, the acquisition can be performed via the terminal unit 5.

### Reference Signs List

- 10: Medical simulator
- 11: Guide control unit
- G: Guide part
- L: Light emitter
- B: Illuminance sensor
- 6: Stomach part
- A: Guide attachment part
- P: Specific site
- 20: Endoscopic system
- 22: Endoscope
- 221: Distal end

## Claims

1. A medical simulator including a hollow organ model whose inside is imaged by an endoscope,
wherein a plurality of guide parts arranged at different specific sites are provided in the hollow organ model.

2. The medical simulator according to claim 1, wherein
each of the guide parts is a light emitter, and
the medical simulator further comprises a guide control unit that controls light emission of the light emitter.

3. The medical simulator according to claim 1, wherein
the hollow organ model is made of a light-transmissive material,
each of the guide parts includes a light emitter and an illuminance sensor attached to an outer surface of the hollow organ model, and
the medical simulator further comprises a guide control unit that controls light emission of the light emitter and light reception of the illuminance sensor.

4. The medical simulator according to claim 2 or 3, wherein the guide part is detachably attached via a guide attachment part provided on the outer surface of the hollow organ model.

5. A training method using a medical simulator including a hollow organ model whose inside is imaged by an endoscope,
wherein a plurality of guide parts arranged at different specific sites are provided in the hollow organ model,
the hollow organ model is made of a light-transmissive material,
the guide part includes a light emitter and an illuminance sensor attached to an outer surface of the hollow organ model,
the light emitter intermittently emits light, and
the illuminance sensor is in a light receivable state when the light emitter is turned off.
